# EUROPÄISCHE PATENTANMELDUNG

(11) **EP 4 516 155 A1**
(43) Veröffentlichungstag der Anmeldung: **05.03.2025**
(21) Anmeldenummer: 23193760.8
(22) Anmeldetag: 28.08.2023
(51) Int. Cl.: A45C 7/00, A45C 13/00, A45C 13/02, A61F 17/00, A45C 5/03, A45C 11/00

(54) **ERSTE-HILFE-KOFFER, VERBANDKASTEN, NOTFALLKOFFER, ERSTE-HILFE-DOPPELKOFFER, NOTFALLDOPPELKOFFER, VERFAHREN ZUR HERSTELLUNG EINES ERSTE-HILFE-KOFFERS, NOTFALLKOFFERS**

(71) Anmelder: W. Söhngen GmbH, 65232 Taunusstein (DE)
(72) Erfinder: MAY, Melanie, 65193 Wiesbaden (DE); ARNOLD, Pascal, 66265 Heusweiler (DE)
(74) Vertreter: Hoffmann, Jürgen

(57) **Zusammenfassung**

Der erfindungsgemäße Notfallkoffer (100, 100') umfasst zwei Kofferschalen (10, 10') und eine Dichtung (20, 20'), wobei die Dichtung (20, 20') von einem Dichtungsmaterial gebildet wird, das stoffschlüssig mit einer der Kofferschalen (10, 10') verbunden ist und die Dichtung (20, 20') im geschlossenen Zustand des Notfallkoffers (100, 100') mit beiden Kofferschalen (10, 10') in Kontakt ist. Vorzugsweise wird die Dichtung (20, 20') unter Zuhilfenahme von Formed-In-Place-Foam-Gasket-Technologie (FIPFG) auf den Notfallkoffer (100, 100') aufgebracht.

## Beschreibung

Die Erfindung betrifft einen Erste-Hilfe-Koffer, Verbandkästen, Notfallkoffer und einen Erste-Hilfe-Doppelkoffer, Notfalldoppelkoffer. Sie betrifft auch ein Verfahren zur Herstellung eines Erste-Hilfe-Koffers, Notfallkoffers.

Unter Notfallkoffer wird hier vorliegend insbesondere verstanden ein Koffer oder Verbandkasten, der befüllt ist mit zumindest einem aus: Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör, sowie Beatmungsmasken. Ein Erste-Hilfe-Koffer ist vorliegend als unter den Begriff des Notfallkoffers im Sinne der Erfindung fallend anzusehen.

Die Erfindung betrifft insbesondere die Ausgestaltung der Dichtung des Erste-Hilfe-Koffers, Verbandkastens oder Notfallkoffers, im Folgenden als Notfallkoffer bezeichnet.

Die Europäische Patentanmeldung mit dem Aktenzeichen EP23152967.8 beschreibt einen Notfallkoffer der aus zwei Notfallkofferhälften besteht.

Es ist Aufgabe der Erfindung, einen Notfallkoffer mit einer verbesserten Dichtung und einen Notfalldoppelkoffer mit verbesserten Dichtungen zu schaffen sowie ein Verfahren anzugeben, mit welchem ein Notfallkoffer mit einer verbesserten Dichtung hergestellt werden kann.

Die Aufgabe wird durch einen Notfallkoffer mit den Merkmalen nach Anspruch 1, durch einen Notfalldoppelkoffer nach Anspruch 12 sowie durch ein Verfahren zur Herstellung eines Notfallkoffers nach Anspruch 13 gelöst.

Der erfindungsgemäße Notfallkoffer umfasst zwei Kofferschalen und eine Dichtung, wobei die Dichtung von einem Dichtungsmaterial gebildet wird, das stoffschlüssig mit einer der Kofferschalen verbunden ist und die Dichtung im geschlossenen Zustand des Notfallkoffers mit beiden Kofferschalen in Kontakt ist.

Das stoffschlüssige Verbinden des Dichtungsmaterials mit der Kofferschale besitzt mehrere Vorteile. Die Gefahr des Loslösens der Dichtung von der Kofferschale, insbesondere die Gefahr des Herausfallens der Dichtung aus einer Aufnahmerille im geöffneten Zustand ist so minimiert und der Notfallkoffer bleibt dicht, auch wenn er bereits mehrfach benutzt wurde. Zudem wird die Dichtigkeit des Notfallkoffers durch die stoffschlüssige Verbindung zwischen Dichtung und Kofferschale im Vergleich zu einem bloßen Einbringen einer fertig geformten Dichtung in eine Aufnahmerille der Kofferschale erhöht. Es kann auf diese Weise eine Dichtigkeit des Innenraums des Notfallkoffers gegenüber der äußeren Umgebung gemäß IP 67 erreicht werden. Es ist in diesem Zusammenhang weiterhin besonders bevorzugt, wenn die korrespondierende Kofferschale einen mechanischen Druck auf die Dichtung ausübt und diese dadurch verformt wird, was die Kontaktfläche zwischen der Dichtung und den Kofferschalen vergrößert und so die Stärke der Abdichtung erhöht. Denkbar ist ebenso, dass an beiden Kofferschalen mindestens eine Dichtung angeordnet ist, die im geschlossenen Zustand des Notfallkoffers jeweils mit der anderen Kofferschale in Kontakt ist, bspw. über hervorstehende Elemente, die an der Kofferschale ausgebildet sind. Ebenso sind sich gegenüberliegende und im geschlossenen Zustand des Notfallkoffers einander gegenseitig kontaktierende Dichtungen denkbar.

Gemäß vorteilhaften Ausführungsformen der Erfindung ist das Dichtungsmaterial ein weichelastischer Schaumstoff. Ein solcher lässt sich besonders leicht und wiederholbar durch den mechanischen Druck, welcher durch die korrespondierende Kofferschale auf die Dichtung ausgeübt wird, verformen und passt sich verschiedenen Geometrien an. Dies vergrößert die Kontaktfläche zwischen der Dichtung und den Kofferschalen in stärkerem Maße, wodurch sich die Stärke der Abdichtung des Innenraums des Notfallkoffers gegenüber der äußeren Umgebung weiter erhöht.

Gemäß einer vorteilhaften Ausführungsform hierzu ist der weichelastische Schaumstoff ein bei Raumtemperatur vernetzter Polyurethanschaum, der aus einer Reaktion eines Polyols oder einer Mischung aus Polyolen und Methylendiphenylisocyanat oder einem seiner Derivate hervorgegangen ist. Eine Dichtung, deren Dichtungsmaterial ein solcher weichelastischer Polyurethanschaum ist, ist besonders günstig, temperaturbeständig, leicht und stabil.

Gemäß vorteilhaften Ausführungsformen bestehen die Kofferschalen aus Acrylnitril-Butadien-Styrol-Kunststoff, wodurch der Notfallkoffer auch bei tiefen Temperaturen steif und zäh bei gleichzeitig hoher Härte bleibt und so der Inhalt des Notfallkoffers besonders gut geschützt ist, da mögliche Fehlfunktionen und Beschädigungen des Notfallkoffers unterbunden werden. Zudem ist ein Acrylnitril-Butadien-Styrol-Kunststoff durch seine Thermoplastizität leicht zu verarbeiten und einer stoffschlüssigen Verbindung mit einem Dichtungsmaterial auf Kunststoffbasis leicht zugänglich. Besonders vorteilhaft ist es in diesem Zusammenhang, wenn das Dichtungsmaterial ein bei Raumtemperatur vernetzter Polyurethanschaum ist, der direkt auf der Kofferschale durch Reaktion eines Polyols oder einer Mischung aus Polyolen und Methylendiphenylisocyanat oder einem seiner Derivate gebildet wurde, da so das Dichtungsmaterial direkt mit der Kofferschale durch chemische Reaktion stoffschlüssig verbunden wird.

Bevorzugt weist ein erfindungsgemäßer Notfallkoffer ein Anpresselement auf, das an einer der Dichtung gegenüberliegenden Kofferschale ausgebildet oder befestigt ist und gleichzeitig derart eingerichtet und positioniert ist, dass es im geschlossenen Zustand des Notfallkoffers mechanischen Druck auf die Dichtung ausübt. Durch den zusätzlichen mechanischen Druck verformt sich die Dichtung noch stärker. Dies vergrößert wiederum die Kontaktfläche zwischen der Dichtung und der Kofferschale noch weiter, wodurch sich die Stärke der Abdichtung des Innenraums des Notfallkoffers gegenüber der äußeren Umgebung noch weiter erhöht. In diesem Zusammenhang ist es besonders bevorzugt, wenn das Anpresselement eine dem Grundriss der Dichtung entsprechende Kontur aufweist, sodass die Dichtung möglichst räumlich durchgängig und von dem Anpresselement mit konstant hohem mechanischem Druck beaufschlagt wird. Besonders vorteilhaft ist es, wenn das Anpresselement eine schmalere Kontur als die Dichtung aufweist, da so das Anpresselement in die Dichtung hineinragen kann und nicht nur die obere, sondern auch die seitlichen Flächen des Anpresselements mit der Dichtung in Kontakt kommen können.

Weist eine der Kofferschalen eine entlang ihres Randes umlaufende Aufnahmerille auf und ist die Dichtung in dieser Aufnahmerille angeordnet, wird die Grenzfläche, über welche die stoffschlüssige Verbindung der Dichtung mit der Kofferschale hergestellt wird, vergrößert und die Stärke der stoffschlüssigen Verbindung erhöht. Dies erhöht die Belastbarkeit der stoffschlüssigen Verbindung und zudem die Dichtigkeit des Notfallkoffers im geschlossenen Zustand. Auch kann in Verbindung mit einem Anpresselement, das eine geringere Breite als die Aufnahmerille aufweist, die Dichtung innerhalb der Aufnahmerille so verformt werden, dass sowohl die Innenflächen der Aufnahmerille als auch die seitlichen Flächen des Anpresselements mit der Dichtung in Kontakt sind.

Gemäß einer vorteilhaften Ausführungsform hierzu ragt die Dichtung über die umlaufende Aufnahmerille hinaus. Hierdurch wird ein Kontakt der Dichtung mit der korrespondierenden Kofferschale erleichtert, da diese bspw. keine dedizierten Anpresselemente aufweisen muss, was die Wahlfreiheit bei der Gestaltung der Geometrie der Kofferschale vergrößert. Bei entsprechender Ausgestaltung der beiden Kofferschalen kann so bspw. auch eine breitere plane Oberfläche der Elemente der korrespondierenden Kofferschale gewählt werden, die in Kontakt mit der Dichtung kommen sollen und dennoch die Dichtung im geschlossenen Zustand von der korrespondierenden Kofferschale mit mechanischem Druck beaufschlagt und entsprechend verformt werden.

Gemäß einer weiteren vorteilhaften Ausführungsform weist eine der Kofferschalen in die Kofferschale steckbare Trennelemente zur Bildung von Untersektionen auf, wobei auf der den steckbaren Trennelementen gegenüberliegenden Kofferschale zusätzliche Dichtungen angeordnet sind, die zumindest in einer Richtung der Kontur der durch die steckbaren Trennelemente bildbaren Untersektionen entsprechen. Dabei sind die zusätzlichen Dichtungen im geschlossenen Zustand des Notfallkoffers mit den Längskanten der Trennelemente in Kontakt. Auf diese Weise können variable Untersektionen durch Umstecken der Trennelemente realisiert werden, wobei die verschiedenen Untersektionen durch das Kontaktieren der Längskanten mit den zusätzlichen Dichtungen im geschlossenen Zustand des Notfallkoffers gegenüber anderen Sektionen abgedichtet sind. Die abgedichteten Untersektion sind dadurch nicht nur gegenüber aus dem Außenbereich stammenden Kontaminationen wie Keimen oder Schmutzpartikeln, sondern auch gegenüber aus anderen Sektionen des Notfallkoffers stammenden Kontaminationen geschützt. Dies können bspw. unerwünschte Wirkstoffe, welche in einer anderen Sektion vorgehalten werden oder Feuchtigkeit sein, welche bspw. aus einer vorgehaltenen Augenspülung stammt. Ein Eintrag solcher Kontaminationen könnte die sichere Funktionsweise von Erste-Hilfe-Materialien oder Erste-Hilfe-Vorrichtungen gefährden, die in der abgedichteten Untersektion vorgehalten werden.

Gemäß einer vorteilhaften Ausführungsform hierzu sind die zusätzliche Dichtungen so angeordnet, dass sie den Konturen der durch die steckbaren Trennelemente bildbaren Untersektionen entsprechen, wobei die zusätzlichen Dichtungen im geschlossenen Zustand des Notfallkoffers mit Längskanten der steckbaren Trennelemente in Kontakt sind. Hier ist insbesondere eine rasterförmige Anordnung der zusätzlichen Dichtungen entlang mehrerer gedachter horizontaler und vertikaler Linien bevorzugt, wobei die Anordnung der zusätzlichen Dichtungen genau dem Raster entspricht, das durch die steckbaren Trennelemente gebildet wird, wenn diese die maximale Anzahl an variablen Untersektionen bilden - bspw. teilen die Linien, welche jeweils beide Raster bilden die Fläche in gleicher Weise in Rechtecke auf. Auf diese Weise ist jede einzelne der variablen Untersektionen, die durch Umstecken der Trennelemente realisiert werden können, abgedichtet. Auch ein nachträgliches Ändern der Untersektionen, bspw. durch den Kunden vor Ort, ist möglich, wobei weiterhin auch jede nachträglich geänderte Untersektion sowohl gegenüber den anderen Untersektionen als auch gegenüber der äußeren Umgebung abgedichtet ist.

Gemäß einer weiteren vorteilhaften Ausführungsform sind an einer der Kofferschalen feste Trennelemente zur Bildung von festen Untersektionen ausgebildet, wobei die Dichtung auf einer Längskante eines der festen Trennelemente angeordnet ist. Bevorzugt weist die Längskante eine Aufnahmerille zur Aufnahme der Dichtung auf und die Dichtung ist in dieser Aufnahmerille angeordnet. Hierdurch wird eine Untersektion des Notfallkoffers gegenüber den anderen Sektionen und der äußeren Umgebung abgedichtet. Diese Untersektion ist dadurch nicht nur gegenüber aus dem Außenbereich stammenden Kontaminationen wie Keimen oder Schmutzpartikeln geschützt, sondern auch gegenüber aus anderen Sektionen des Notfallkoffers stammenden Kontaminationen.

Gemäß einer weiteren vorteilhaften Ausführungsform sind an einer der Kofferschalen feste Trennelemente zur Bildung von festen Untersektionen ausgebildet, wobei eine zusätzliche Dichtung auf einer Längskante eines der festen Trennelemente angeordnet und stoffschlüssig mit der Längskante verbunden ist. Bevorzugt weist in diesem Zusammenhang die Längskante eine Aufnahmerille auf und die Dichtung ist in dieser Aufnahmerille angeordnet. Die zusätzliche Dichtung erzeugt auf diese Weise eine zusätzliche Abdichtung, welche die feste Untersektion des Notfallkoffers zusätzlich gegenüber der äußeren Umgebung abdichtet, während diese weiterhin gegenüber dem Rest des Innenbereichs abgedichtet ist. Hierdurch ist die feste Untersektion doppelt gegenüber aus dem Außenbereich stammenden Kontaminationen geschützt, während ein Schutz gegenüber aus anderen Sektionen des Notfallkoffers stammenden Kontaminationen aufrecht erhalten bleibt.

Ist der Notfallkoffer mit zumindest einem aus Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör oder Beatmungsmasken befüllt, können diese leicht zugänglich und gleichzeitig geschützt vor Kontaminationen vorgehalten werden.

Der erfindungsgemäße Notfalldoppelkoffer weist zwei erfindungsgemäße Notfallkoffer auf, die lösbar miteinander verbunden sind, wobei jeweils eine Kofferschale zur Herstellung eines geschlossenen Notfallkoffers an der korrespondierenden Kofferschale desselben Notfallkoffers arretierbar ist. Auf diese Weise können die beiden Notfallkofferhälften verbunden bleiben, während die Arretierung der Kofferschalen an der jeweils korrespondierenden Kofferschale eines der Notfallkoffer gewährleistet, dass die Dichtungen im geschlossenen Zustand mit beiden Kofferschalen in Kontakt sind und der Inhalt der einzelnen Notfallkoffer vor Kontamination geschützt ist. Sind beide Notfallkoffer zu einem Notfalldoppelkoffer verbunden, hat der behandelnde Ersthelfer, Arzt oder sonstiges Bedienpersonal einen großen Notfallkoffer mit viel Material. Bei gleicher Bestückung der Notfallkoffer sind dann etwa besonders viele Verbandsmaterialien vorhanden. Bei unterschiedlicher Bestückung der Notfallkoffer können sich die Inhalte einander ergänzen. Wenn die Notfallkoffer getrennt werden, bleibt der verbesserte Schutz vor Kontaminationen weiterhin gewährleistet und die Notfallkoffer können von zwei verschiedenen Bedienpersonen verwendet werden. Das Auftrennen kann gegebenenfalls schnell vor Ort erfolgen. Bei Vorhandensein eines Notfalldoppelkoffers können somit zwei Bedienpersonen jeweils einen Notfallkoffer verwenden und entsprechend zwei Patienten mit zuvor vor Kontamination geschützten Erste-Hilfe-Materialien gleichzeitig behandeln.

Das erfindungsgemäße Verfahren zur Herstellung eines Notfallkoffers beinhaltet das Bereitstellen zweier Kofferschalen und das Auftragen einer fließfähigen Dichtungskomponente auf eine der Kofferschalen, wobei die fließfähige Dichtungskomponente so gewählt ist, das sie nach dem Auftragen zum einen mit der Oberfläche der Kofferschale eine chemische Bindung eingeht und zum anderen zu einem festen Dichtungsmaterial reagiert, wodurch das entstandene Dichtungsmaterial stoffschlüssig mit der Kofferschale verbunden ist und eine Dichtung bildet. Ferner beinhaltet das erfindungsgemäße Verfahren das Verbinden der beiden Kofferschalen zu einem Notfallkoffer in einer Weise, dass die Dichtung im geschlossenen Zustand des Notfallkoffers mit beiden Kofferschalen in Kontakt ist.
In diesem Zusammenhang erfolgt das Auftragen der fließfähigen Dichtungskomponente bevorzugt maschinell. Das Einbringen von Keimen durch einen Menschen während des manuellen Einsetzens eine bereits fertig geformten Dichtung wird so ausgeschlossen, was die Keimbelastung des Innenraums des Notfallkoffers senkt. Dies ist insbesondere in Bezug auf die spätere Anwendung des Notfallkoffers von Vorteil. Auch die Gefahr des ungenauen bzw. fehlerhaften Einfügens einer fertig geformten Dichtung durch menschliche Ungenauigkeiten bzw. Fehler wird ausgeschlossen. Ein solcher Fehler kann bspw. in einem Knicken der Dichtung bestehen, was zu Undichtigkeiten führen kann. Zudem müssen keine verschiedenen Größen und Formen von Dichtungen vorgehalten werden, um verschiedene Notfallkoffer produzieren zu können. Darüber hinaus wird der Zeitaufwand für das Einbringen der Dichtung verringert, wobei der zeitliche Aufwand, der durch das im Stand der Technik verwendete manuelle Einsetzen der Dichtung verursacht wird, etwa 2 Minuten pro Notfallkoffer beträgt. Überdies werden Fertigungstoleranzen besser ausgeglichen, da sich die fließfähige Dichtungskomponente nach dem Auftragen und vor dem Ausbilden des Dichtungsmaterials selbst nivelliert, was in einer gleichmäßigeren Geometrie und insbesondere Höhe der resultierenden Dichtung auf der Kofferschale resultiert. Bevorzugt wird in dem Verfahren eine fließfähige Dichtungskomponente eingesetzt, die bei Raumtemperatur zu einem Dichtungsmaterial reagiert, das zu einer gleichförmigen Dichtung aufschäumt. Denkbar ist in diesem Zusammenhang, dass eine chemische Reaktion zwischen zwei Bestandteilen der fließfähigen Dichtungskomponente das Aufschäumen initiiert. Dies kann bspw. dadurch realisiert werden, dass die einzelnen Bestandteile der Dichtungskomponente erst unmittelbar vor dem Moment des Auftragens miteinander vermischt werden und die entsprechende chemische Reaktion durch das Vermischen initiiert wird. Mit dem erfindungsgemäßen Verfahren kann eine ansatzlos geschlossene Dichtung realisiert werden, die unter Berücksichtigung einer gegebenen maximalen Auftragungsdicke praktisch jede denkbare Kontur und Geometrie aufweisen kann. Auch wenn einige der genannten Vorteile auf maschinelles Auftragen der fließfähigen Dichtungskomponente zurückzuführen sind, ist ein manuelles Auftragen ebenso denkbar und explizit von der Erfindung eingeschlossen. Auch hierdurch ergeben sich gegenüber dem Stand der Technik bereits mehrere der oben genannten Vorteile, wie bspw. das Ausgleichen von Fertigungstoleranzen, die starke stoffschlüssige Verbindung und die Möglichkeit, verschiedene Notfallkoffer produzieren zu können, ohne verschiedene Dichtungen vorhalten zu müssen.

Gemäß einer vorteilhaften Ausführungsform hierzu weist eine der Kofferschalen eine entlang des Randes umlaufende Aufnahmerille auf und die fließfähige Dichtungskomponente wird innerhalb der Aufnahmerille aufgetragen. Auf diese Weise wird ein Fließen der fließfähigen Dichtungskomponente an unerwünschte Stellen der Kofferschale vermieden. Dies führt insbesondere in Verbindung mit der Selbstnivellierung der Dichtungskomponente zu einer besonders gleichmäßigen Geometrie und Höhe der gebildeten Dichtung.

Bestehen die Kofferschalen aus Acrylnitril-Butadien-Styrol-Kunststoff und enthält die fließfähige Dichtungskomponente sowohl ein Polyol oder eine Mischung aus Polyolen als auch Methylendiphenylisocyanat oder eines seiner Derivate, so kann ein bereits bei Raumtemperatur vernetzender Polyurethanschaum erhalten werden, der äußerst stabil gegen Einflüssen wie Feuchtigkeit, Staub und Temperatur ist.

Nachfolgend wird die Erfindung unter Bezug auf die jeweilige Zeichnung näher beschrieben, wobei
- Fig. 1: eine perspektivische Ansicht einer ersten Ausführungsform des erfindungsgemäßen Notfallkoffers unter Weglassen der Dichtung zeigt;
- Fig. 1b: eine perspektivische Ansicht des Notfallkoffers aus Fig. 1 mit Dichtung zeigt;
- Fig. 1c: eine perspektivische Ansicht des Notfallkoffers aus den Figuren 1 und 1b in geschlossenem Zustand zeigt;
- Fig. 2: eine perspektivische Ansicht einer zweiten Ausführungsform des Notfallkoffers mit variablen Untersektionen zeigt.
- Fig. 3: eine perspektivische Ansicht einer dritten Ausführungsform des erfindungsgemäßen Notfallkoffers unter Weglassen der Dichtung zeigt;
- Fig. 3b: eine perspektivische Ansicht des Notfallkoffers aus Fig. 3 mit Dichtung zeigt;
- Fig. 4: eine perspektivische Ansicht einer Ausführungsform des erfindungsgemäßen Notfalldoppelkoffers unter Weglassen der Dichtung zeigt;
- Fig. 4b: eine perspektivische Ansicht des Notfalldoppelkoffers aus Fig. 4 mit Dichtung zeigt;
- Fig. 4c: eine perspektivische Ansicht des Notfallkoffers aus den Figuren 4 und 4b in geschlossenem Zustand zeigt;
- Fig. 5: in perspektivischer Ansicht das Auftragen der fließfähigen Dichtungskomponente in einer Ausführungsform des erfindungsgemäßen Verfahrens.

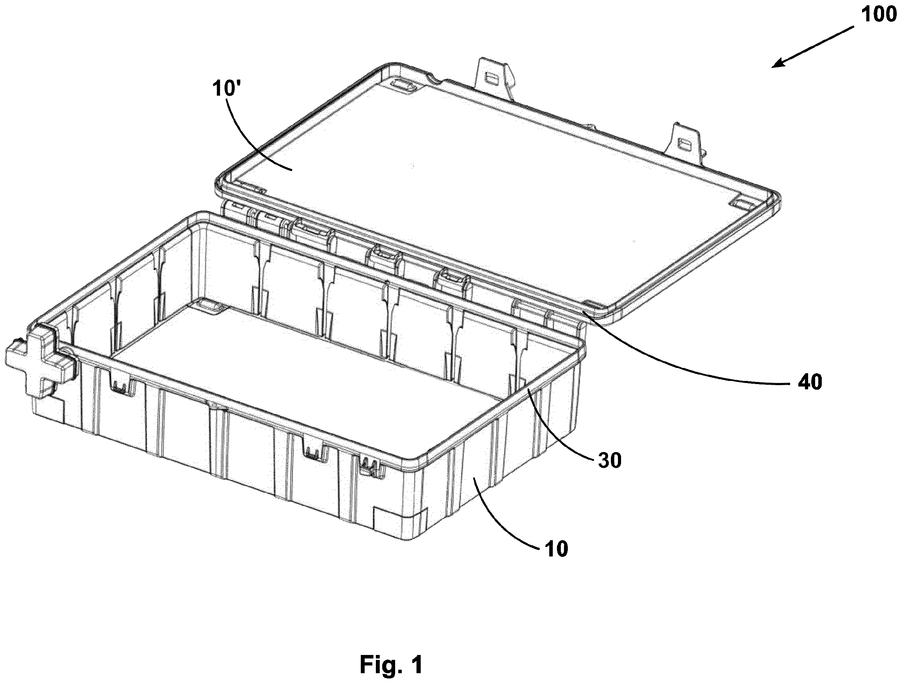

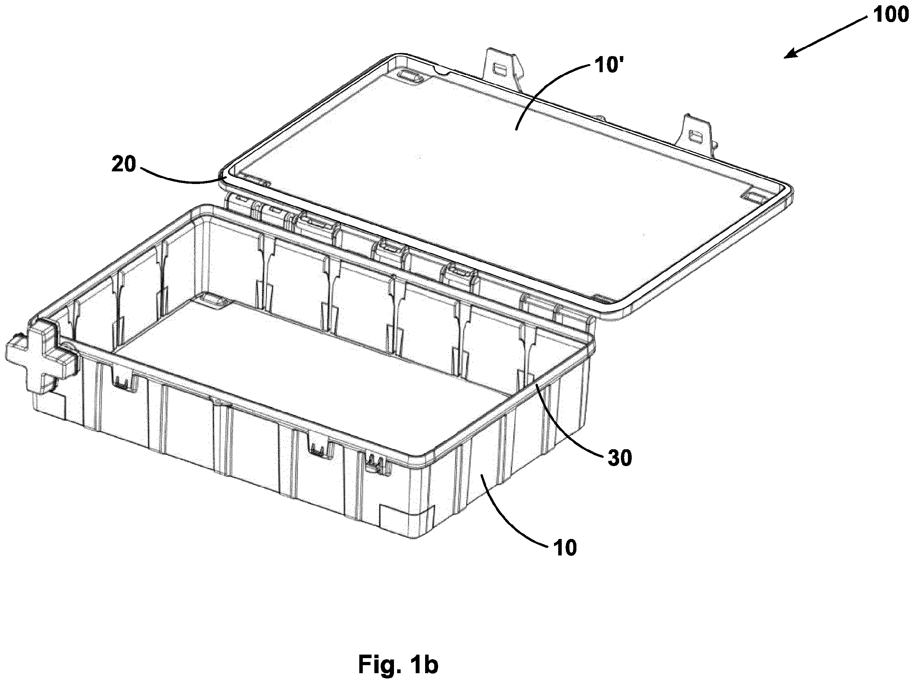

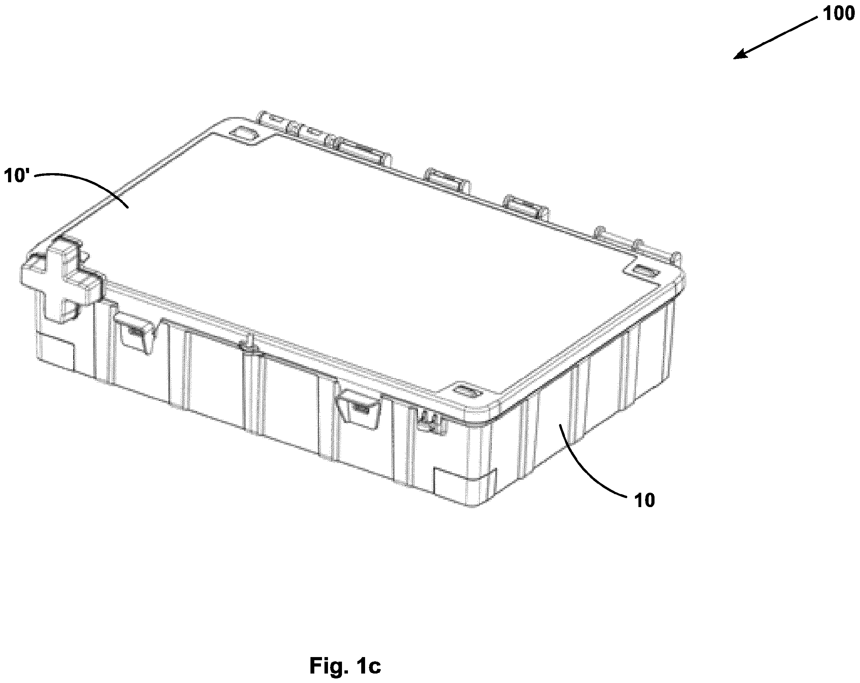

In Fig. 1 ist ein im Ganzen mit 100 bezeichneter erfindungsgemäßer Notfallkoffer dargestellt, der zwei Kofferschalen 10 und 10' umfasst. Der besseren Übersicht halber ist die Dichtung 20, welche ein erfindungsgemäßer Notfallkoffer 100 aufweist, nicht in Fig. 1 dargestellt (vgl. Fig. 1b für eine entsprechende Darstellung des gleichen Notfallkoffers 100 mit Dichtung 20). Die Kofferschalen 10 und 10' sind in der hier dargestellten Ausführungsform nicht gleichartig. Die im Wesentlichen in der oberen Hälfte befindliche Kofferschale 10' ist flach ausgebildet und dient in erster Linie als Deckel. Die untere Kofferschale 10 weist eine deutlich ausgeprägte Vertiefung auf und dient der Aufnahme von Erste-Hilfe-Materialien und/oder Erste-Hilfe-Vorrichtungen, wie Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör, Beatmungsmasken und Ähnlichem.
Entlang des Randes der oberen Kofferschale 10' ist eine umlaufende Aufnahmerille angeordnet, welche der Aufnahme der Dichtung 20 (nicht in Fig. 1 dargestellt) dient. Diese ist innerhalb der Aufnahmerille 40 angeordnet und stoffschlüssig mit der Kofferschale 10' verbunden. Im geschlossenen Zustand des Notfallkoffers 100 (vgl. Fig. 1c für eine entsprechende Darstellung) sind beide Kofferschalen 10 und 10' mit der Dichtung 20 in Kontakt. Hierdurch wird der Innenraum des Notfallkoffers 100 gegenüber der äußeren Umgebung abgedichtet und so das Kontaminationsrisiko für den Inhalt des Notfallkoffers 100 durch den Eintritt von bspw. Keimen oder Schmutzpartikeln aus dem Außenbereich vermindert. Auch die Möglichkeit des Eintritts von Feuchtigkeit aus dem Außenbereich, welche die sichere Funktionsweise von Erste-Hilfe-Materialien oder Erste-Hilfe-Vorrichtungen gefährden kann, wird so minimiert.
Um die Stärke der Abdichtung zu erhöhen, ist am Rand der Kofferschale 10 umlaufend ein Anpresselement 30 angeordnet, welches im geschlossenen Zustand des Notfallkoffers 100 einen mechanischen Druck auf die Dichtung 20 (nicht in Fig. 1 dargestellt) ausübt. Zu diesem Zweck sind das Anpresselement 30 und die Aufnahmerille 40 in der hier dargestellten Ausführungsform so ausgebildet und relativ zueinander angeordnet, dass das Anpresselement 30 im geschlossenen Zustand des Notfallkoffers 100 in die Aufnahmerille 40 hineinragt. Abweichend von dem hier gezeigten beispielhaften Ausführungsform ist ebenso denkbar, dass die Dichtung 20 über die Aufnahmerille 40 hinausragt und das Anpresselement 30 im geschlossenen Zustand des Notfallkoffers 100 entsprechend mechanischen Druck auf die Dichtung 20 ausübt, ohne dass das Anpresselement 30 in die Aufnahmerille 40 hineinragt. Durch die dadurch hervorgerufene Verformung der Dichtung 20 vergrößert sich die Kontaktfläche zwischen der Dichtung 20 und der Notfallkofferhälfte 10, wodurch die Stärke der Abdichtung des Innenraums des Notfallkoffers 100 gegenüber der äußeren Umgebung erhöht wird. Diese Wirkung wird in besonders starken Maße erzielt, wenn das Anpresselement 30, wie oben ausgeführt, im geschlossenen Zustand des Notfallkoffers 100 in die Aufnahmerille 40 hineinragt.
Eine beispielhaftes besonders vorteilhaftes Vorgehen, die stoffschlüssige Verbindung des Dichtungsmaterials mit der Kofferschale 10' zu realisieren, wobei ein erfindungsgemäßer Notfallkoffer 100 explizit nicht darauf eingeschränkt ist, unter Zuhilfenahme dieses Vorgehens hergestellt worden zu sein, besteht in einem maschinellen Auftragen einer fließfähigen Dichtungskomponente auf die Kofferschale 10', wobei die fließfähige Dichtungskomponente mit der Oberfläche der Kofferschale 10' eine chemische Bindung eingeht und selbst zu dem Dichtungsmaterial reagiert, um eine Dichtung 20 zu bilden. Dieses beispielhafte Vorgehen kann der *Formed-In-Place-Foam-Gasket-*Technologie (FIPFG) zugeordnet werden.

In Fig. 1b ist der gleiche Notfallkoffer 100 wie in Fig. 1 dargestellt, wobei in der in Fig. 1b gezeigten Darstellung die Dichtung 20 zu sehen ist, die innerhalb der Aufnahmerille 40 (in Fig. 1b nicht zu sehen, da durch Dichtung 20 verdeckt) angeordnet und mit der Kofferschale 10' stoffschlüssig verbunden ist.
Es ist erkennbar, dass das Anpresselement 30 im geschlossenen Zustand des Notfallkoffers 100 mechanischen Druck auf die Dichtung 20 ausübt, indem es in die Dichtung 20 hineinragt. Hierdurch wird der Innenraum des Notfallkoffers 100 im geschlossenen Zustand noch stärker gegenüber der äußeren Umgebung abgedichtet als dies ohne Anpresselement 30 der Fall wäre, da so die Dichtung 20 bloß in leichtem Kontakt mit der Kofferschale 10 wäre.

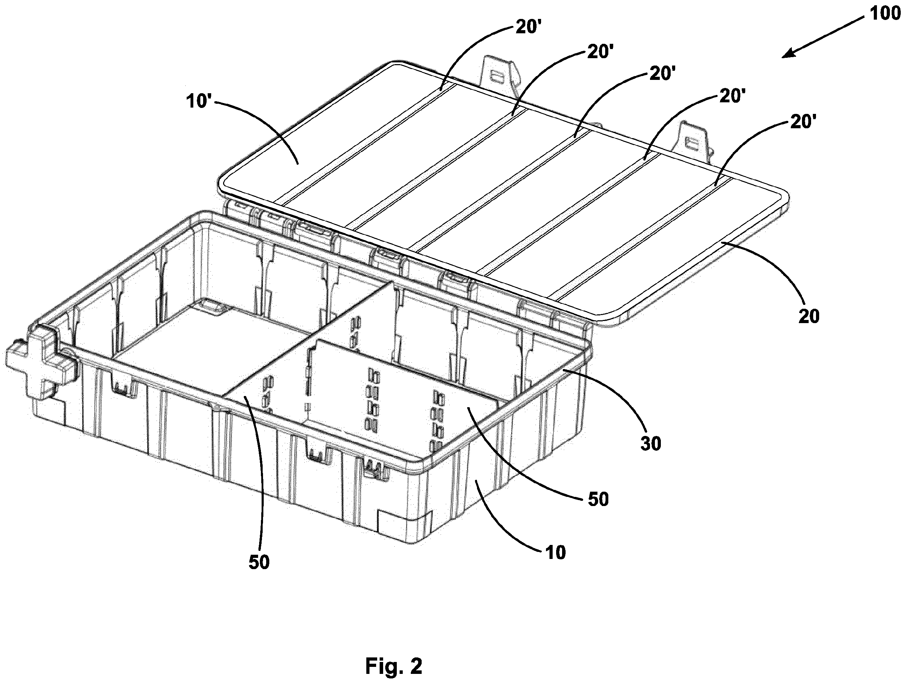

Fig. 1c, welche den Notfallkoffer 100 aus den Figuren 1 und 2 in geschlossenem Zustand zeigt, lässt erkennen, dass die Kofferschalen 10 und 10' einen im Wesentlichen gleichen Grundriss aufweisen, wobei die Kofferschale 10 und 10' mit Hilfe von Klemmlaschen und Rastnasen miteinander verrasten. Durch dieses Verrasten wird der mechanische Druck auf die Dichtung 20 (nicht in Fig. 1c dargestellt) durch das Anpresselement 30 der Kofferschale 10 aufrecht erhalten und der Innenraum des Notfallkoffers 100 im geschlossenen Zustand gegenüber der äußeren Umgebung zuverlässig abgedichtet.

Fig. 2 veranschaulicht die Möglichkeit zur variablen Bildung von Untersektionen durch in die Kofferschale 10 steckbare Trennelemente 50. Auf der den steckbaren Trennelementen 50 gegenüberliegenden Kofferschale 10' sind mehrere Dichtungen 20' angeordnet, welche in dieser beispielhaften Darstellung quer zur Längsachse des Notfallkoffers 100 ausgerichtet sind. Auf diese Weise können durch entsprechendes Anordnen der steckbaren Trennelemente 50 eine Vielzahl von abgedichteten Untersektionen geschaffen werden. Die Dichtungen 20' kontaktieren dabei die Längskanten der quer zur Längsachse des Notfallkoffers 100 ausgerichteten steckbaren Trennelemente 50. Die Untersektionen werden so gegenüber aus dem Außenbereich stammenden Kontaminationen wie Keimen oder Schmutzpartikeln geschützt. Auch gegenüber aus anderen Sektionen des Notfallkoffers 100 stammenden Kontaminationen sind diese abgedichteten Untersektionen geschützt. Der hier gezeigte Notfallkoffer 100 weist zudem ein Anpresselement 30 am oberen Rand der Kofferschale 10 sowie eine umlaufende Dichtung 20 an der Kofferschale 10' auf. Analog der Beschreibung zu Fig. 1 übt das Anpresselement 30 im geschlossenen Zustand des Notfallkoffers 100 entsprechend mechanischen Druck auf die Dichtung 20 aus, wodurch die Stärke der Abdichtung des Innenraums des Notfallkoffers 100 gegenüber der äußeren Umgebung erhöht wird. Alternativ oder zusätzlich zur hier gezeigten beispielhaften Ausführungsform können zusätzliche Dichtungen 20' derart an der Kofferschale 10' angeordnet sein, dass die Längskanten derjenigen steckbaren Trennelemente 50 durch die Dichtungen 20' kontaktiert werden, die parallel zur Längsachse des Notfallkoffers 100 ausgerichtet sind, sobald dieser geschlossen ist.

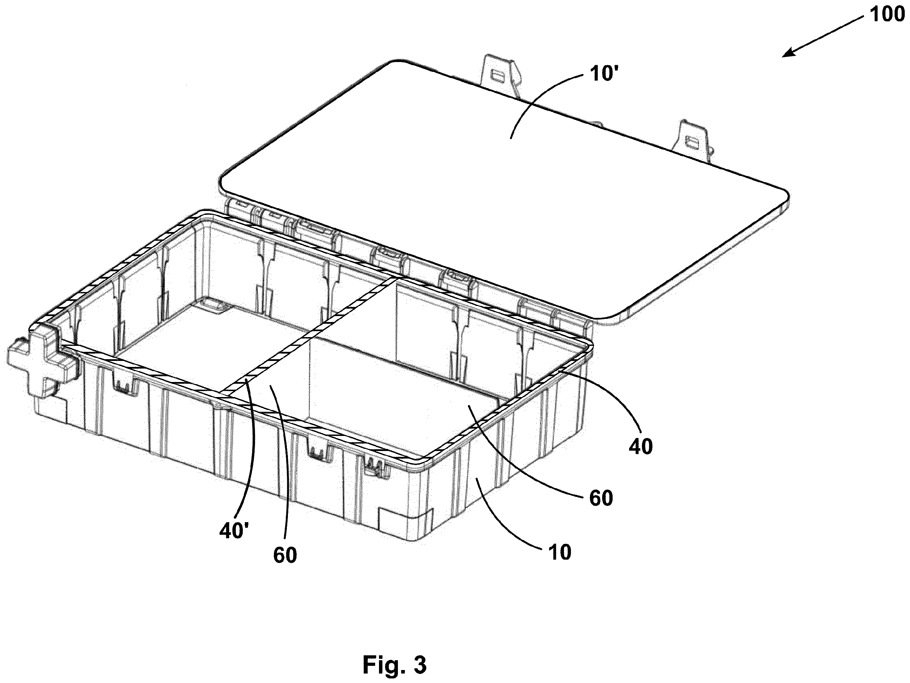

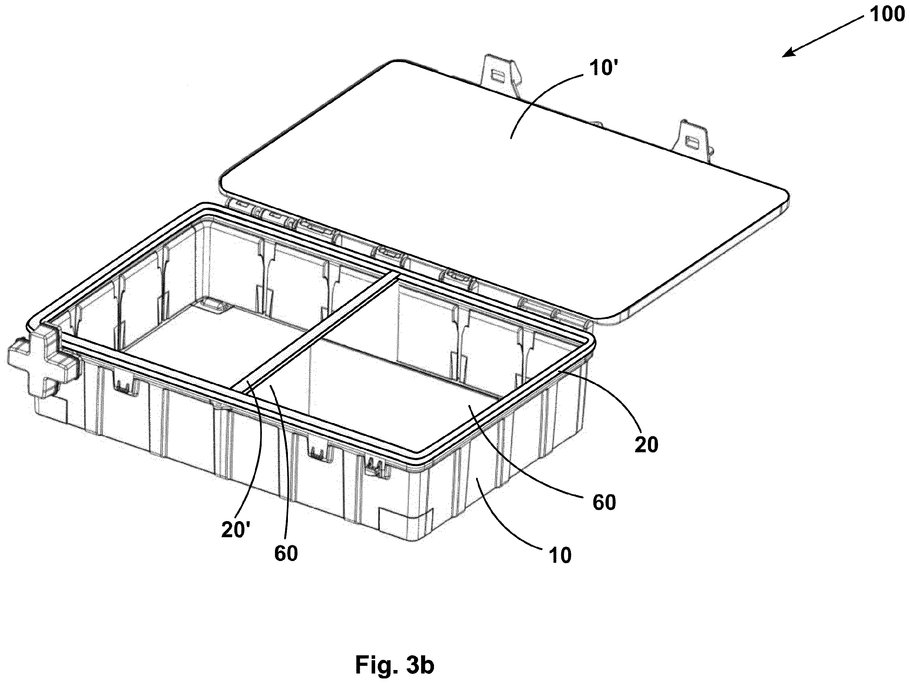

In Fig. 3 ist eine zweite Ausführungsform eines im Ganzen mit 100 bezeichneten erfindungsgemäßen Notfallkoffers dargestellt, bei der an der Kofferschale 10 feste Trennelemente 60 mit nach oben weisenden Längskanten zur Bildung von festen Untersektionen ausgebildet sind. Der besseren Übersicht halber ist die Dichtung 20, welche ein erfindungsgemäßer Notfallkoffer 100 aufweist, nicht in Fig. 3 dargestellt (vgl. Fig. 3b für eine entsprechende Darstellung des gleichen Notfallkoffers 100 mit Dichtung 20). Die festen Untersektionen, welche durch die festen Trennelemente 60 gebildet werden dienen der getrennten Aufbewahrung von Erste-Hilfe-Materialien und/oder Erste-Hilfe-Vorrichtungen. Zudem weist die Längskante eines der festen Trennelemente 60 eine Aufnahmerille 40' zur Aufnahme einer Dichtung 20' (nicht in Fig. 3 gezeigt) auf. Anstelle eines Anpresselements 30 weist die Kofferschale 10 in der hier gezeigten Ausführungsform am oberen Rand eine umlaufende Aufnahmerille 40 zur Aufnahme einer Dichtung 20 auf.
Auch weist die hier dargestellte Kofferschale 10' im Gegensatz zum Notfallkoffer 100 aus den Figuren 1 bis 1c keine Aufnahmerille 40 auf. Die in Richtung des Innenraums des Notfallkoffers 100 weisende Oberfläche der Kofferschale 10' ist vielmehr im Wesentlichen plan, um im geschlossenen Zustand des Notfallkoffers 100 in Kontakt mit den Dichtungen 20, 20' (nicht in Fig. 3 dargestellt) zu gelangen, die in den Aufnahmerillen 40 und 40' angeordnet sind. Alternativ oder zusätzlich dazu könnte die Kofferschale 10' analog der Kofferschale 10 des in den Fig. 1-2b dargestellten Notfallkoffers 100 ein oder mehrere Anpresselemente 30 aufweisen, welche im geschlossenen Zustand des Notfallkoffers 100 in die Aufnahmerillen 40 und 40' hineinragen. Dieser Umstand verdeutlicht, dass die Anordnung der Aufnahmerille 40, 40' bzw. des Anpresselements 30 oder einer Mehrzahl der beiden explizit nicht an eine konkrete Kofferschale 10 bzw. 10' gebunden ist, sondern beide Elemente in sich gegenüberliegender Weise an entsprechenden Kofferschalen 10, 10' angeordnet sein können.

Fig. 3b veranschaulicht die Ausgestaltung der erfindungsgemäßen Ausführungsform des Notfallkoffers 100, die in Fig. 3 dargestellt ist, wobei in Fig. 3b die Dichtung 20 gezeigt ist. Die Dichtung 20 ist innerhalb der Aufnahmerille 40 (nicht in Fig. 3b dargestellt) am oberen Rand der Kofferschale 10 angeordnet, mit der Kofferschale 10 stoffschlüssig verbunden und ragt nach oben über die Aufnahmerille 40 hinaus. In analoger Weise ist die Dichtung 20' innerhalb der Aufnahmerille 40' (nicht in Fig. 3b dargestellt) auf der Längskante eines der festen Trennelemente 60 angeordnet, mit diesem stoffschlüssig verbunden und ragt in zur Dichtung 20 analoger Weise über die Aufnahmerille 40' hinaus. Durch das Hinausragen der Dichtungen 20, 20' nach oben über die Aufnahmerillen 40, 40' hinaus gelangt die plane Oberfläche der Kofferschale 10' im geschlossenen Zustand des Notfallkoffers 100 mit den Dichtungen 20, 20' in Kontakt und übt zudem einen mechanischen Druck auf diese aus, was die Stärke der Abdichtung des Innenraums des Notfallkoffers 100 gegenüber der äußeren Umgebung erhöht. Die zusätzliche Dichtung 20' erzeugt auf diese Weise eine gegenüber dem Rest des Innenbereichs abgedichtete Untersektion des Notfallkoffers 100.

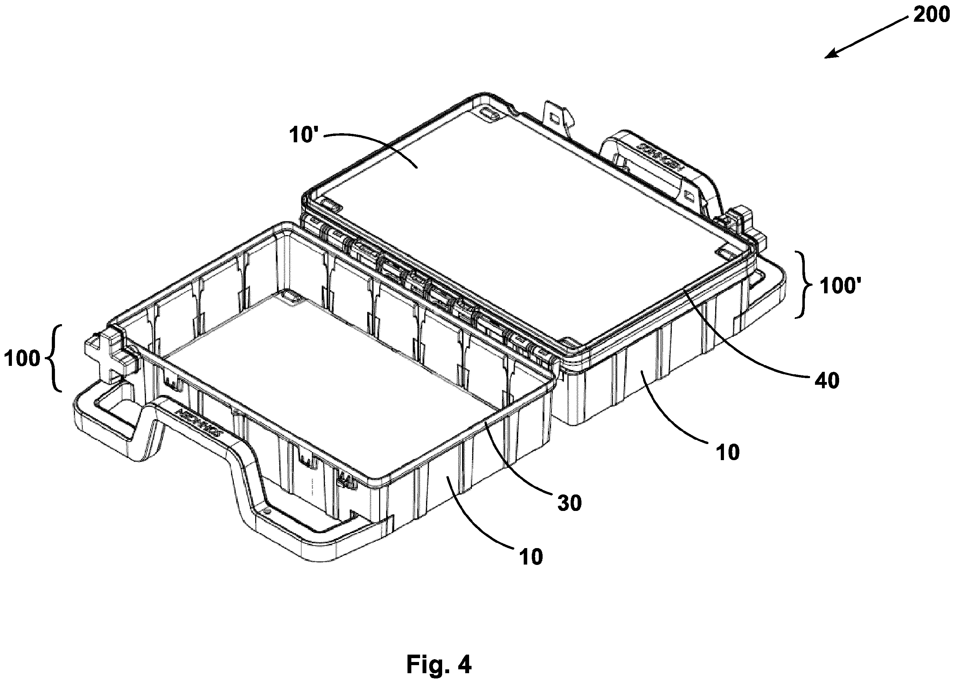

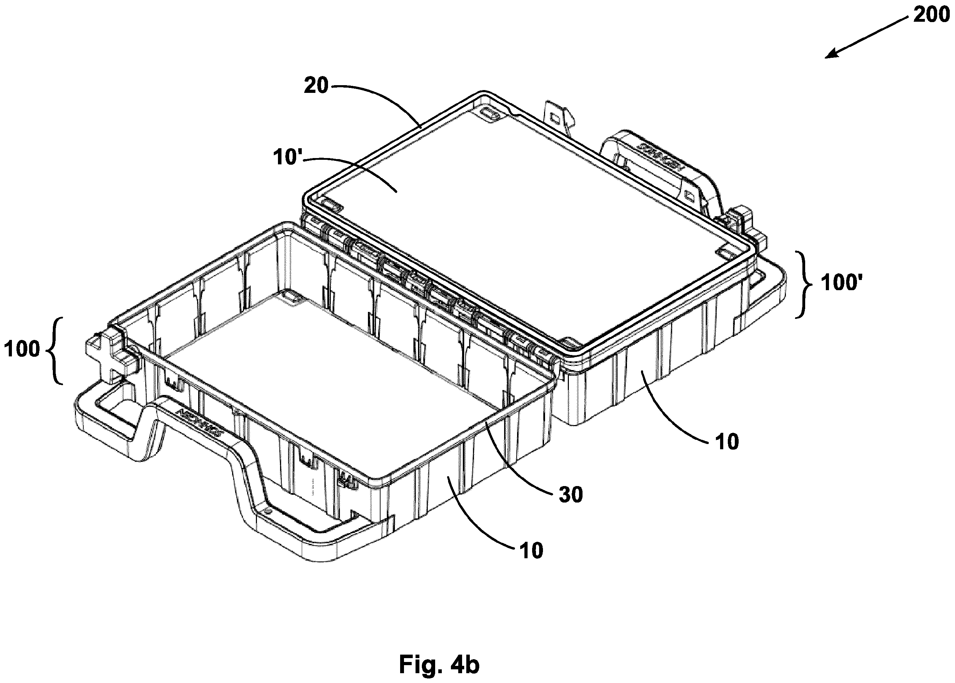

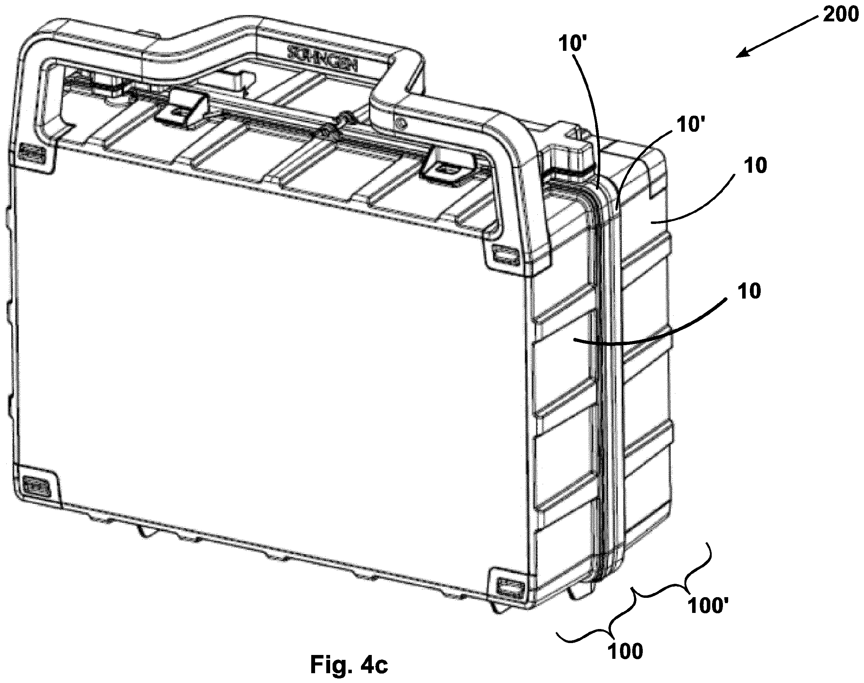

In Fig. 4 ist ein im Ganzen mit 200 bezeichneter erfindungsgemäßer Notfalldoppelkoffer dargestellt, der zwei erfindungsgemäße Notfallkoffer 100, 100' umfasst. Die Notfallkoffer 100, 100' weisen dabei jeweils den gleichen Aufbau wie der in den Figuren 1 bis 1c dargestellte Notfallkoffer 100 auf, wobei der besseren Übersicht halber die Dichtung 20 nicht dargestellt ist (vgl. Fig. 4b für eine entsprechende Darstellung mit Dichtung 20). Beim geöffneten Notfallkoffer 100, der im Wesentlichen in der linken Hälfte von Fig. 4 gezeigt ist, ist dabei das Anpresselement 30 an der Kofferschale 10 zu sehen. An der Kofferschale 10' ist die Aufnahmerille 40 zur Aufnahme der Dichtung 20 (nicht dargestellt) angeordnet. Die Notfallkoffer 100 und 100' sind über zueinander komplementäre Steckvorrichtungen, die an oberen Kante der der jeweils anderen Kofferschale 100 bzw. 100' zugewandten Seite angeordnet sind, lösbar miteinander verbunden. Dabei ist jeweils eine Kofferschale 10' zur Herstellung eines geschlossenen Notfallkoffers 100, 100' an der korrespondierenden Kofferschale 10 desselben Notfallkoffers 100, 100' arretierbar.

Fig. 4b veranschaulicht die Ausgestaltung der erfindungsgemäßen Ausführungsform des Notfalldoppelkoffers 200 aus Fig. 4, wobei in Fig. 4b die Dichtung 20 gezeigt ist. Die Dichtung 20 ist innerhalb der am Rand der Kofferschale 10' umlaufenden Aufnahmerille 40 (nicht in Fig. 4b dargestellt) angeordnet und mit der Kofferschale 10' stoffschlüssig verbunden. Im geschlossenen Zustand ist die Dichtung 20 mit der Kofferschale 10 in Kontakt, wobei das Anpresselement 30 einen zusätzlichen mechanischen Druck auf die Dichtung 20 ausübt und so die Stärke der Abdichtung des jeweiligen Innenraums der Notfallkoffer 100, 100' gegenüber der äußeren Umgebung erhöht. Analoges gilt für den Notfallkoffer 100', dessen innerer Aufbau in Fig. 4b durch die aufgeklappte Kofferschale 10' verdeckt ist.

Fig. 4c, welche den Notfalldoppelkoffer 200 aus den Figuren 4 und 4b in geschlossenem Zustand zeigt, verdeutlicht, dass die Notfallkoffer 100, 100' im Wesentlichen gleich gestaltet sind. Durch die Arretierung der Kofferschalen 10' an der jeweils korrespondierenden Kofferschale 10 zur Herstellung eines geschlossenen Notfallkoffers 100, 100' ist gewährleistet, dass die Dichtungen 20 jeweils mit den korrespondierenden Kofferschalen 10 in Kontakt sind, wobei durch die Anpresselemente 30 (nicht dargestellt) ein konstanter zusätzlicher mechanischer Druck auf die Dichtungen 20 (nicht dargestellt) wirkt und so die Stärke der Abdichtung des jeweiligen Innenraums der Notfallkoffer 100, 100' gegenüber der äußeren Umgebung erhöht wird.

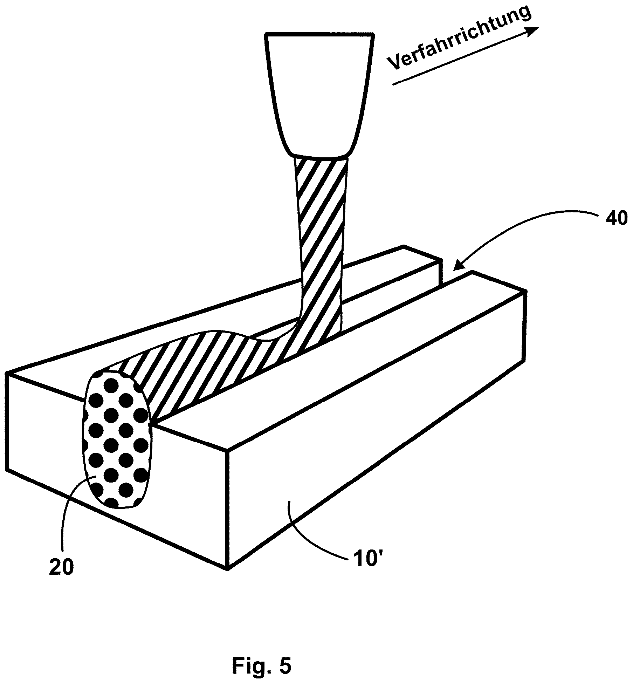

Fig. 5 zeigt eine Momentaufnahme des Auftragens der fließfähigen Dichtungskomponente, wie es gemäß einer Ausführungsform des erfindungsgemäßen Verfahrens erfolgen kann. Das Auftragen erfolgt hier durch einen Applikator, der am oberen Rand von Fig. 5 gezeigt ist. Aus diesem fließt eine Dichtungskomponente in eine Aufnahmerille 40, die an der Kofferschale 10' angeordnet ist, während der Applikator entlang der Aufnahmerille 40 in der mit dem von "Verfahrrichtung" überschriebenen Pfeil am oberen Rand von Fig. 5 angegebenen Richtung verfahren wird. Das Dosieren der fließfähigen Dichtungskomponente und das Entlangführen entlang der Aufnahmerille 40 erfolgt dabei bevorzugt maschinell. Die fließfähige Dichtungskomponente geht mit der Oberfläche der Kofferschale eine chemische Bindung ein und reagiert unter Aufschäumen zu einem Dichtungsmaterial, das eine Dichtung 20 bildet, während der Applikator weiter entlang der Aufnahmerille 40 verfahren wird, sodass das Dichtungsmaterial durch die zeitliche Verzögerung des Aufschäumens nicht mit dem Applikator in Kontakt kommt. Die Dichtung 20 ragt in dieser beispielhaften Darstellung über die Aufnahmerille 40 hinaus.

Zwar wurde die Erfindung unter Bezug auf bestimmte Ausführungsformen beschrieben, es sollte sich aber verstehen, dass die Erfindung auf diese Beispiele nicht beschränkt ist, und dass zahlreiche Abwandlungen vom Fachmann vorgesehen und erdacht werden können, nachdem er sich die Beispiele angesehen hat.

### Bezugszeichenliste

- 100: Notfallkoffer
- 100': Notfallkoffer
- 10: Kofferschale
- 10': Kofferschale
- 20: Dichtung
- 20': Dichtung
- 30: Anpresselement
- 40: Aufnahmerille
- 40': Aufnahmerille
- 50: steckbares Trennelement
- 60: festes Trennelement
- 200: Notfalldoppelkoffer

## Patentansprüche

1. Notfallkoffer (100, 100'), mit zwei Kofferschalen (10, 10') und einer Dichtung (20, 20'), **dadurch gekennzeichnet, dass** die Dichtung (20, 20') von einem Dichtungsmaterial gebildet wird, wobei das Dichtungsmaterial stoffschlüssig mit einer der Kofferschalen (10, 10') verbunden ist, und wobei die Dichtung (20, 20') im geschlossenen Zustand des Notfallkoffers (100, 100'), mit beiden Kofferschalen (10, 10') in Kontakt ist.

2. Notfallkoffer (100, 100') nach Anspruch 1, **dadurch gekennzeichnet, dass** das Dichtungsmaterial ein weichelastischer Schaumstoff ist.

3. Notfallkoffer (100, 100') nach Anspruch 2, **dadurch gekennzeichnet, dass** der weichelastische Schaumstoff ein bei Raumtemperatur vernetzter Polyurethanschaum ist, der aus einer Reaktion eines Polyols oder einer Mischung aus Polyolen und Methylendiphenylisocyanat oder einem seiner Derivate hervorgegangen ist.

4. Notfallkoffer (100, 100') nach einem der vorhergehenden Ansprüche, **dadurch gekennzeichnet, dass** die Kofferschalen (10, 10') aus Acrylnitril-Butadien-Styrol-Kunststoff bestehen.

5. Notfallkoffer (100, 100') nach einem der vorhergehenden Ansprüche, mit einem Anpresselement (30), wobei das Anpresselement (30) an einer der Dichtung (20) gegenüberliegenden Kofferschale (10, 10') ausgebildet oder befestigt ist, und wobei das Anpresselement (30) derart eingerichtet und positioniert ist, dass es im geschlossenen Zustand des Notfallkoffers (100, 100') mechanischen Druck auf die Dichtung (20, 20') ausübt.

6. Notfallkoffer (100, 100') nach einem der vorhergehenden Ansprüche, **dadurch gekennzeichnet, dass** eine der Kofferschalen (10, 10') eine entlang ihres Randes umlaufende Aufnahmerille (40, 40') aufweist und die Dichtung (20, 20') in dieser Aufnahmerille (40, 40') angeordnet ist.

7. Notfallkoffer (100, 100') nach Anspruch 6, **dadurch gekennzeichnet, dass** die Dichtung (20, 20') über die umlaufende Aufnahmerille (40, 40') hinausragt.

8. Notfallkoffer (100, 100') nach einem der vorhergehenden Ansprüche, **dadurch gekennzeichnet, dass** eine der Kofferschalen (10, 10') in die Kofferschale (10, 10') steckbare Trennelemente (50) zur Bildung von Untersektionen aufweist, und wobei auf der den steckbaren Trennelementen (50) gegenüberliegenden Kofferschale (10, 10') zusätzliche Dichtungen (20, 20') angeordnet sind, die zumindest in einer Richtung der Kontur der durch die steckbaren Trennelemente (50) bildbaren Untersektionen entsprechen, wobei die zusätzlichen Dichtungen (20, 20') im geschlossenen Zustand des Notfallkoffers (100, 100') mit Längskanten der steckbaren Trennelemente (50) in Kontakt sind.

9. Notfallkoffer (100, 100') nach Anspruch 8, **dadurch gekennzeichnet, dass** die zusätzliche Dichtungen (20, 20') so angeordnet sind, dass sie den Konturen der durch die steckbaren Trennelemente (50) bildbaren Untersektionen entsprechen, wobei die zusätzlichen Dichtungen (20, 20') im geschlossenen Zustand des Notfallkoffers (100, 100') mit Längskanten der steckbaren Trennelemente (50) in Kontakt sind.

10. Notfallkoffer (100, 100') nach einem der Ansprüche 1 bis 5 oder 8 bis 9, **dadurch gekennzeichnet, dass** an einer der Kofferschalen (10, 10') feste Trennelemente (60) zur Bildung von Untersektionen ausgebildet sind, wobei die Dichtung (20, 20') auf einer Längskante eines der festen Trennelemente (60) angeordnet ist, wobei bevorzugterweise die Längskante eine Aufnahmerille (40, 40') aufweist und die Dichtung (20, 20') in dieser Aufnahmerille angeordnet ist.

11. Notfallkoffer (100, 100') nach einem der Ansprüche 1 bis 7, **dadurch gekennzeichnet, dass** an einer der Kofferschalen (10, 10') feste Trennelemente (60) zur Bildung von festen Untersektionen ausgebildet sind, wobei eine zusätzliche Dichtung (20, 20') auf einer Längskante eines der festen Trennelemente (60) angeordnet und stoffschlüssig mit der Längskante verbunden ist, wobei bevorzugterweise die Längskante eine Aufnahmerille (40, 40') aufweist und die Dichtung (20, 20') in dieser Aufnahmerille (40, 40') angeordnet ist.

12. Notfallkoffer (100, 100') nach einem der vorhergehenden Ansprüche, **dadurch gekennzeichnet, dass** der Notfallkoffer (100, 100') befüllt ist mit zumindest einem aus:
- Pflaster und Verbandsmaterial,
- Einmalhandschuhen,
- Erste-Hilfe-Scheren und Pinzetten,
- Hygienemundschutz,
- Hygienekleidung,
- Notduschen und Augenspülung,
- Desinfektionsmittel und Spender dazu,
- Defibrillatoren und Zubehör,
- Beatmungsmasken.

13. Notfalldoppelkoffer, mit zwei Notfallkoffern (100, 100') nach einem der vorhergehenden Ansprüche, **dadurch gekennzeichnet, dass** die beiden Notfallkoffer (100, 100') lösbar miteinander verbunden sind, wobei jeweils eine Kofferschale (10, 10') zur Herstellung eines geschlossenen Notfallkoffers (100, 100') an der korrespondierenden Kofferschale (10, 10') desselben Notfallkoffers (100, 100') arretierbar ist.

14. Verfahren zur Herstellung eines Notfallkoffers (100, 100'), das aufweist:
- Bereitstellen zweier Kofferschalen (10, 10');
- Auftragen einer fließfähigen Dichtungskomponente auf eine Kofferschale (10, 10'), wobei die fließfähige Dichtungskomponente so gewählt ist, das sie nach dem Auftragen zum einen mit der Oberfläche der Kofferschale (10, 10') eine chemische Bindung eingeht und zum anderen zu einem festen Dichtungsmaterial reagiert, wodurch das entstandene Dichtungsmaterial stoffschlüssig mit der Kofferschale (10, 10') verbunden ist und eine Dichtung (20, 20') bildet;
- Verbinden der beiden Kofferschalen (10, 10') zu einem Notfallkoffer (100, 100') in einer Weise, dass die Dichtung (20, 20') im geschlossenen Zustand des Notfallkoffers (100, 100') mit beiden Kofferschalen (10, 10') in Kontakt ist.

15. Verfahren nach Anspruch 14, wobei eine der Kofferschalen (10, 10') eine entlang des Randes umlaufende Aufnahmerille (40, 40') aufweist und wobei die fließfähige Dichtungskomponente innerhalb der Aufnahmerille (40, 40') aufgetragen wird.

16. Verfahren nach einem der Ansprüche 14 oder 15, wobei die Kofferschalen (10, 10') aus Acrylnitril-Butadien-Styrol-Kunststoff bestehen und die fließfähige Dichtungskomponente sowohl ein Polyol oder eine Mischung aus Polyolen als auch Methylendiphenylisocyanat oder eines seiner Derivate enthält.
